# EUROPEAN PATENT SPECIFICATION

(11) **EP 4 633 461 B1**
(45) Date of publication and mention of the grant of the patent: **18.11.2026**
(21) Application number: 23825434.6
(22) Date of filing: 08.12.2023
(51) Int. Cl.: A61B 5/024, A61B 5/11, A61B 5/00, A61B 5/021, A61B 5/02, A61B 5/026

(54) **MONITORING DEVICE**
ÜBERWACHUNGSEINRICHTUNG
DISPOSITIF DE SURVEILLANCE

(30) Priority: 16.12.2022 GB 202219033
(43) Date of publication of application: 22.10.2025
(73) Proprietor: M G Electric (Colchester) Ltd, Colchester Essex CO4 9HY (GB)
(72) Inventor: SHIRVANI, Hassan, Chelmsford Essex CM3 4SS (GB); MOHAGHEGH, Vahaj, Colchester Essex CO4 9HY (GB)
(74) Representative: Marks & Clerk LLP
(86) International application number: PCT/GB2023/053175
(87) International publication number: WO 2024/126984

(56) References cited:
- EP-B1- 3 269 299
- WO-A1-2019/217778
- WO-A1-2022/035430
- US-A1- 2003 135 124
- US-A1- 2006 079 782
- US-B2- 11 406 274

## Description

### Technical field

The invention relates to a blood flow monitoring device.

### Background

Anticipated human lifespan is getting longer and overall, the impacts of modern life on blood vessel ailments is becoming more evident and prevalent inside the maturing blood vessel network of the body. These ailments may frequently appear as stenoses (an irregular narrowing or blockage inside the blood vessel). Stenoses may cause back pressure in, for example, the arterial veins, the iliac and femoral conduits and/or the inside carotid corridors, bringing about angina, claudication or stroke.

An arteriovenous fistula (AVF) is an irregular connection between an artery and a vein, where blood flows directly from the artery to the vein, bypassing capillaries. Small AVFs may be surgically created for use in dialysis for patients with severe kidney disease. Such AVFs need to be continually monitored, for example, for stenoses.

Current methods of monitoring AVFs include using ultrasound techniques, such as Doppler ultrasound/ultrasound dilution, which use ultrasound to produce an image, from which a professional can estimate the blood flow speed through the blood vessels. Whether a stenoses is present and the extent of the stenoses may then be determined based on the estimated blood flow speed. Typically however, the patient is required to attend multiple appointments with the professional so that the ultrasound test may be performed and the results analysed.

More recently, Magnetic Resonance Imaging (MRI) has be used to determine blood flow speed and stream rates, from which the presence and extent of stenoses may be determined. However this is a very expensive method and again, requires the patient to attend multiple appointments with a professional.

There exists a need for improved methods of monitoring blood flow through a blood vessel, and in particular, for monitoring AVFs.

EP 3 269 299 B1 relates to a blood pressure measuring device including: a pressure measuring tool; a fastening member configured to fasten the pressure measuring tool onto a skin region covering an anastomotic region of a shunt blood vessel; and a control circuit configured to receive an input signal from the pressure measuring tool and to process the input signal to calculate a pressure inside the anastomotic region of the shunt blood vessel.

US 2006/079782 A1 relates to a real-time signal processing technique for ultrasonic imaging of tissue vibrations for localizing the source of a bruit in 2D image with respect to the anatomy and/or for obtaining simultaneous information about vibrations and the underlying blood flow.

WO 2022/035430 A1 relates to a device for measuring blood flow through a blood vessel, which includes a conductive elastomer having a variable resistance.

WO 2019/217778 A1 relates to a method of generating a signal representing vibrations originating from a blood vessel of a subject and decomposing the signal into one or more first intrinsic oscillatory modes and one or more second intrinsic oscillatory modes. The first intrinsic oscillatory modes are used to determine a blood volume status of the subject, and the second intrinsic oscillatory modes are used to determine mechanical properties of the blood vessel.

US 11,406,274 B2 relates to remotely acquiring sensor data from a wearable patch mounted on a patient.

US 2003/135124 A1 relates to measuring cardiac output, limb blood flow, perfusion, blood pressure, artery elasticity, and cardiovascular deterioration and disease, including performing these measurements on a continuous heartbeat-by-beat basis.

### Summary

According to the invention, there is provided a blood flow monitoring device as recited by claim 1. The device comprises a sensor configured to be placed against skin of a user and to collect data indicative of movement of the skin caused by blood flow through a blood vessel in a region under the skin, wherein an output of the sensor comprises a waveform comprising a series of peaks and valleys; and a controller comprising: a blood flow parameter determiner and a blood flow impairment determiner. The blood flow parameter determiner is configured to determine, based on the waveform output by the sensor, a systolic blood pressure, a diastolic blood pressure, a maximum diameter of the blood vessel and a minimum diameter of the blood vessel. The systolic blood pressure is determined based on an average height of the peaks of the waveform from the x-axis. The diastolic blood pressure is determined based on an average height of the valleys of the waveform from the x-axis. The maximum diameter of the blood vessel is determined based on the average height of the peaks from a signal mean of the waveform. The minimum diameter of the blood vessel is determined based on the average height of the valleys from the signal mean of the waveform. The blood flow impairment determiner is configured to determine a degree of stenosis and a blood flow volume based on the determined maximum diameter of the blood vessel, the determined minimum diameter of the blood vessel, the determined systolic blood pressure and the determined diastolic blood pressure.

Optionally, the sensor comprises a sensing element configured to deform on movement of the skin of the user, and wherein deformation of the sensing element changes an electrical property of the sensor.

Optionally, the sensor comprises a strain gauge.

Optionally, the data indicative of movement of the skin of the user comprises data indicative of strain applied to the strain gauge by movement of the skin.

Optionally, the blood flow monitoring device comprises a plurality of sensors configured to be placed against the skin of the user, wherein each sensor is configured to collect data indicative of movement of the skin.

Optionally, the blood flow impairment determiner is further configured to determine an extent of blood flow impairment based on the degree of stenosis and the blood flow volume.

Optionally, the blood flow impairment determiner is configured to compare the degree of stenosis to a stenosis threshold and the blood flow volume to a blood flow volume threshold to determine the extent of the blood flow impairment.

Optionally, the blood flow impairment determiner is configured to determine a risk level comprising one of a high risk, a moderate risk and a low risk, based on the comparison of the degree of stenosis with the stenosis threshold and the comparison of the blood flow volume with the blood flow volume threshold.

Optionally, the blood flow monitoring device further comprises an indicator configured to provide an indication to the user indicating whether the blood flow through the blood vessel is impaired.

Also disclosed herein, but not claimed, is a method of determining whether blood flow through a blood vessel is impaired, the method comprising: collecting, by a sensor placed against skin of a user, data indicative of movement of the skin caused by blood flow through a blood vessel in a region under the skin; determining, by a blood flow parameter determiner, a blood flow parameter based on the data collected by the sensor, and determining, by a blood flow impairment determiner, whether blood flow through the blood vessel is impaired based on the calculated blood flow parameter.

Optionally, the blood flow parameter determiner determines a maximum diameter of the blood vessel and a minimum diameter of the blood vessel based on the data collected by the sensor.

Optionally, the blood flow parameter determiner determines a systolic blood pressure and a diastolic blood pressure based on the data collected by the sensor.

Optionally, the blood flow impairment determiner determines a degree of stenosis and a blood flow volume based on one or more of: the determined maximum diameter of the blood vessel, the determined minimum diameter of the blood vessel, the determined systolic blood pressure and the determined diastolic blood pressure.

Optionally, the method further comprises determining, by the blood flow impairment determiner, an extent of blood flow impairment based on the degree of stenosis and the blood flow volume.

Optionally, the blood flow impairment determiner compares the degree of stenosis to a stenosis threshold and the blood flow volume to a blood flow volume threshold to determine the extent of the blood flow impairment.

Optionally, the method further comprises determining, by the blood flow impairment determiner, a risk level comprising one of a high risk, a moderate risk and a low risk, based on the comparison of the degree of stenosis with the stenosis threshold and the comparison of the blood flow volume with the blood flow volume threshold.

Optionally, the method further comprises providing an indication to the user indicating whether the blood flow through the blood vessel is impaired.

### Brief description of the drawings

Figure 1 shows an exemplary blood flow monitoring device;
Figure 2 shows an exemplary controller of a blood flow monitoring device;
Figure 3 shows a flow diagram illustrating an exemplary method of using the exemplary blood flow monitoring device to determine whether blood flow through a blood vessel is impaired;
Figure 4 shows an exemplary signal output of a sensor of an exemplary blood flow monitoring device;
Figure 5 shows an exemplary Fast Fourier Transform (FFT) spectrogram;
Figure 6 shows a schematic of a blood vessel including a stenotic region; and
Figure 7 shows a series of exemplary signal outputs of an exemplary blood flow monitoring device.

### Detailed description

Generally disclosed herein are blood flow monitoring devices comprising a sensor configured to be placed against skin of a user to collect data indicative of movement of the skin caused by blood flow through a blood vessel in a region under the skin. In exemplary arrangements, the sensor may comprise a strain gauge, such as a resistor based strain gauge or an FBG strain sensor, and the data collected by the sensor may comprise strain data indicative of the strain applied to the sensor as a result of movement of the skin. A blood flow parameter may be calculated based on the data collected by the sensor. For example, the blood flow parameter may comprise a parameter indicative of the maximum and minimum blood vessel diameter, and/or a parameter indicative of systolic and/or diastolic blood pressure. Based on the calculated blood flow parameter(s), a determination is made indicating whether blood flow through the blood vessel is impaired. A blood vessel may be impaired due to an irregular narrowing or blockage inside the blood vessel, which may be caused by a stenosis. Based on the calculated blood flow parameter(s), it may be determined whether a stenosis is present and/or the extent of the stenosis.

Advantageously, the blood flow monitoring device described herein provides a non-invasive and cost-effective method of monitoring blood flow through a blood vessel, and in particular an AVF. Furthermore, the patient is able to perform the test themselves, without the need to attend an appointment with a professional. In exemplary arrangements, the blood flow monitoring device may provide an indication to a user, for example via a display, of the extent of the stenosis and/or whether the user needs to follow up with a professional.

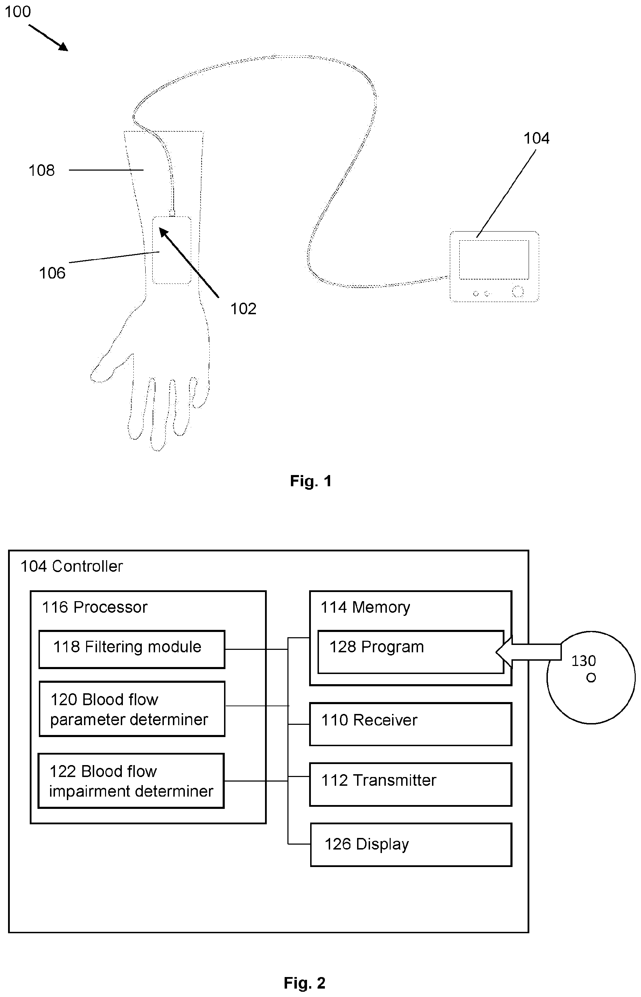

Figure 1 shows an exemplary blood flow monitoring device 100. The blood flow monitoring device 100 comprises a sensor 102 and a controller 104.

The sensor 102 may comprise any sensor capable of detecting movement of skin 108 of a user when placed thereupon. Figure 1 shows the sensor 102 placed upon the skin 108 of an arm of a user, however the skilled person will appreciate that this is merely an example and the sensor is placed on the skin of the user over a blood vessel of interest (which may or may not be located in the user's arm). At least a portion of the sensor 102 is configured to deform, or otherwise change shape, with movement of the skin against which it is placed. A property of the sensor 102, which may be an electrical property, changes with the deformation, and as such, data indicative of movement of the skin of the user may be collected by monitoring the change in the property of the sensor 102.

In the exemplary monitoring device 100, the sensor 102 comprises a strain gauge. Exemplary strain gauges may comprise a linear strain gauge. Exemplary strain gauges may have a rated resistance of substantially 350 Ohm (optionally ± 0.3%), and a gauge factor of substantially 2.05 (optionally ± 1.01%). Exemplary strain gauges are those provided by HBM, such as the LY Linear Strain Gauges.

As will be known to the skilled person a strain gauge is configured to measure the strain and/or force on an item/object/surface against which it is placed. As the strain gauge deforms, or otherwise changes shape with the item/material upon which it is placed, a property of the strain gauge changes. For example, the strain gauge may comprise a sensing element comprising an electrical conductor, and the resistance of the electrical conductor may be configured to change as the strain gauge, and therefore the sensing element, is deformed or otherwise changes shape. When placed against the skin 108 of the user, the sensing element of the strain gauge deforms with movement of the skin 108, and the strain gauge is therefore able to collect data indicative of movement of the skin of the user, which may at least partially be caused by blood flow through a blood vessel under the skin 108. The skilled person will be aware of the operation and capabilities of strain gauges and further detail is not given here.

As described above, in the exemplary monitoring device 100, the sensor 102 comprises a resistor-based strain gauge. In alternative arrangements, the sensor 102 may comprise alternative sensors configured to collect data indicative of movement of the skin. For example, the sensor 102 may comprise Fiber Bragg Gratings (FBG) optical or other optical technology strain gauges, or else stretch sensors, which may be resistance or capacitance based. The skilled person will be able to envisage further sensors capable of detecting movement of the skin of the user.

In the exemplary arrangement of Figure 1, the monitoring device 100 includes a sensor pad 106 comprising the sensor 102. The sensor pad 106 comprises a plurality of sensors 102, however the skilled person will appreciate that in alternative arrangements, a single sensor 102 may be provided. The sensor pad 106 may be formed of a flexible sheet, or flexible sheets, of material such that deformation of the sensor 102 coupled thereto, with movement of the user's skin 108, is not prevented.

The monitoring device 100 may comprise a retention portion configured to retain the sensor pad 106, and therefore the sensor(s) 102, in contact with the skin 108 of the user. In the exemplary arrangement shown in Figure 1, the retention portion comprises an adhesive portion located on the sensor pad 106 and which is configured to adhere to the skin 108 of the user when placed thereon. In alternative arrangements, no retention portion may be provided and the sensor pad 106 and/or sensor 102 may simply be placed against the skin 108. In further arrangements, the retention portion may comprise a cuff comprising the sensor 102, or straps, or clips etc. The skilled person will be able to envisage further ways of retaining the sensor 102 in contact with the skin 108 of the user.

The monitoring device 100 further comprises a controller 104. The controller 104 may be in data communication with the sensor 102.

In the exemplary monitoring device 100 shown in Figure 1, the connection between the sensor 102 and the controller 104 is shown as a wired connection, however the skilled person will appreciate that in alternative arrangements, the connection may be wireless.

An exemplary controller 104 is shown in Figure 2.

The controller 104 comprises a receiver 110 and may also comprise a transmitter 112. The receiver 110 and/or transmitter 112 may be in data communication with other entities, such as the sensor 102, servers/hubs and/or functions in a telecommunications network and are configured to transmit and receive data accordingly.

The controller 104 may further comprise a memory 114, a processor 116, a filtering module 118, a blood flow parameter determiner 120, a blood flow impairment determiner 122 and a display 126. The display 126 may comprise a display screen. The memory 114 may comprise a non-volatile memory and/or a volatile memory. The memory 114 may have a computer program 128 stored therein. The computer program 128 may comprise instructions for performing the methods disclosed herein. The computer program 128 may be loaded in the memory 114 from a non-transitory computer readable medium 130, on which the computer program is stored. The processor 116 is configured by the computer program 128 to perform one or more of the functions of the filtering module 118, the blood flow parameter determiner 120, and the blood flow impairment determiner 122, as set out below.

Each of the receiver 110, transmitter 112, memory 114, processor 116, filtering module 118, blood flow parameter determiner 120, blood flow impairment determiner 122 and display 126, is in data communication with the other components 110, 112, 114, 116, 118, 120, 122, 126 of the controller 104. The controller 104 can be implemented as a combination of computer hardware and software. In particular, filtering module 118, blood flow parameter determiner 120, and blood flow impairment determiner 122 may be implemented as software configured to run on the processor 116. The memory 114 stores the various programs/executable files that are implemented by a processor 116, and also provides a storage unit for any required data. The programs/executable files stored in the memory 114, and implemented by the processor 116, can include the filtering module 118, blood flow parameter determiner 120, and blood flow impairment determiner 122, but are not limited to such.

Use of the blood flow monitoring device 100 to determine whether blood flow through a blood vessel is impaired (that is, irregularly narrowed) will now be described with reference to Figures 1-6.

When a user wishes to use the blood flow monitoring device 100, the sensor 102 is placed against the skin 108 of the user in the vicinity of the blood vessel, or portion of the blood vessel, to be monitored. Ideally, the sensor 102 is placed on the skin of the user such that the sensor 102 is located directly above the blood vessel, or portion of the blood vessel, to be monitored.

In one particular application, the blood flow monitoring device 100 may be configured to provide an indication as to whether blood flow through an AVF of the user is impaired. In such applications, the user would place the sensor 102 against the skin in a region located above the AVF (ideally directly above the AVF). The skilled person will appreciate that the blood flow monitoring device 100 may be used to provide an indication as to whether blood flow through substantially any blood vessel is impaired in a similar way as described herein, however for the purposes of this particular example, use of the blood flow monitoring device 100 to determine whether blood flow through an AVF is impaired is described.

The retention portion of the blood flow monitoring device 100 may be used to retain the sensor 102 in contact with the skin of the user. In the exemplary arrangement shown in Figure 1, the sensor pad 106 is pushed against the skin 108 of the user such that the adhesive portion of the sensor pad 106 adheres to the skin 108 of the user. As such, the sensor 102 is retained in position on the skin 108 of the user, and above the blood vessel/AVF. In alternative arrangements, in which no retention portion is present, the sensor 102 may simply be placed upon the skin 108 of the user.

302: Once placed on the skin 108 of the user, the sensor 102 collects data indicative of movement of the skin 108 of the user.

In the exemplary blood flow monitoring device 100, the sensor 102 comprises a strain gauge configured to collect data indicative of the movement of the skin 108 of the user. The strain gauge is configured to measure strain, as will be explained below. As such, in the exemplary blood flow monitoring device 100 the sensor 102 collects data indicative of strain applied to the strain gauge caused by movement of the skin 108 of the user.

As the skin of the user moves, due to blood flowing through the blood vessel/AVF, the sensing element of the strain gauge deforms, which causes the resistance of the sensing element of the strain gauge to change in proportion to the amount of strain being applied to the strain gauge. Based on the measured change in resistance, strain may be determined, as explained below.

Strain, *ε*, is defined as the ratio of the change in length of a material to the original, unaffected length: $ϵ = \frac{Δ L}{L}$

Each strain gauge has a gauge factor, GF, which is the ratio between the between the fractional change in electrical resistance ( $\frac{Δ R}{R}$) and the fractional change in length ( $\frac{Δ L}{L}$) of the strain gauge. The gauge factor is a constant and is provided by the manufacturer. As such, strain, ε, may be determined based on the change in resistance measured by the strain gauge as below: $GF = \frac{\frac{Δ R}{R}}{\frac{Δ L}{L}} = \frac{\frac{Δ R}{R}}{ϵ}$

The sensor 102 collects data for a predetermined period of time. In exemplary arrangements, the predetermined time period may be one of: substantially 30 seconds, substantially 45 seconds, substantially 60 seconds or substantially 90 seconds, although the skilled person will appreciate that alternative time periods may be used. In exemplary arrangements, the predetermined time period comprise a range, such as one of: substantially 30 seconds to substantially 45 seconds, substantially 30 seconds to substantially 60 seconds, substantially 45 seconds to substantially 60 seconds, substantially 45 seconds to substantially 90 seconds, although the skilled person will again appreciate that alternative ranges may be used.

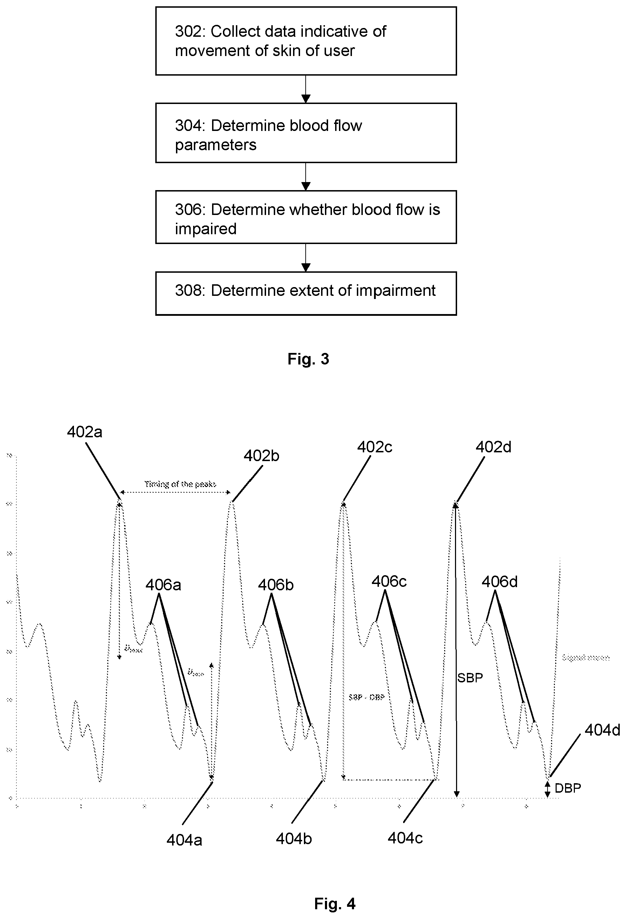

Figure 4 shows a plot of the output of the sensor 102, when placed against the skin of a user over a blood vessel such as an AVF. The plot shows the strain measured by the strain gauge on the y-axis and time on the x-axis. As can be seen from Figure 4, the output of the sensor 102 comprises a waveform comprising a series of peaks 402a-d and valleys 404a-d. Between each of the peaks 402a-d and valleys 404a-d, there are a series of intermediate peaks 406a-d (see for example, intermediate peaks 406b between peak 402b and valley 404b). Similar outputs (i.e. waveforms) to those shown in Figure 4 may be produced when using alternative sensors 102, such as Fiber Bragg Gratings (FBG) optical or other optical technology strain gauges, or else stretch sensors

In exemplary arrangements, the raw data collected by the sensor 102 may be filtered by the filtering module 118 of the controller 104. The filtering module 118 may use a filter, such as the Butterworth filter, to smooth the waveform output of the sensor before determination of the blood flow parameters, as described below. Figure 4 shows a signal output that has been filtered by the filtering module 118.

304: The blood flow parameter determiner 120 of the controller 104 determines one or more blood flow parameters based on the data collected by the sensor 102.

The blood flow parameters may comprise one or more of: a maximum diameter (*dₘₐₓ*) of the blood vessel over which the sensor 102 is placed, a minimum diameter (*dₘᵢₙ*) of the blood vessel over which the sensor 102 is placed, a systolic blood pressure (SBP), a diastolic blood pressure (DBP), a heart rate (*ω*), and an estimated diameter of the stenotic portion (d) of the blood vessel over which the sensor 102 is placed.

The blood flow parameters are determined from the signal output of the sensor 102 as shown in Figure 4. That is:
- The maximum diameter of the blood vessel over which the sensor 102 is placed (*dₘₐₓ*) can be determined based on the average height of the signal peaks 402a-d from the signal mean (i.e. from y = signal mean)..
- The minimum diameter of the blood vessel over which the sensor 102 is placed (*dₘᵢₙ*) can be determined based on the average height of the signal valleys 404a-d from the signal mean..
- The systolic blood pressure (SBP) can be determined based on the average height of the signal peaks 402a-d from the x-axis (i.e. from y=0).
- The diastolic blood pressure (DBP) can be determined based on the average height of the signal valleys 404a-d from the x-axis (i.e from y=0).
- The heart rate (*ω*) may be determined based on the time interval between signal peaks 402a-d divided by the number of signal peaks.
- An estimated diameter of the stenotic portion (d) can be determined based on the signal mean (the signal mean may also be referred to as the signal running mean or signal moving average).

The blood flow parameters may be determined by the blood flow parameter determiner 120 using a peak detection algorithm or alternative methods, as will be familiar to the skilled person.

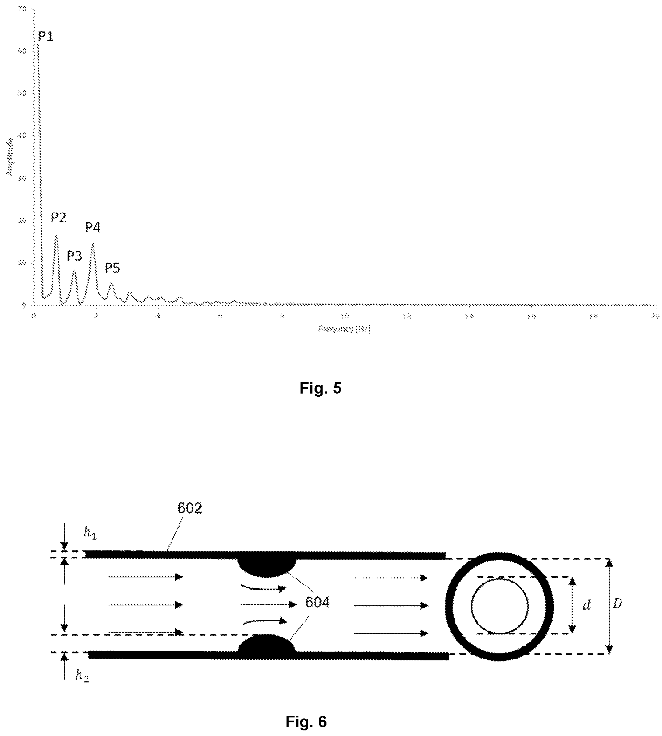

The blood flow parameter determiner 120 may alternatively, or additionally, determine the systolic and diastolic blood pressure by applying a Fast Fourier Transform (FFT) to the signal output of the sensor 102 to produce an FFT spectrogram of the signal output. Figure 5 shows an FFT spectrogram of the signal output of Figure 4. The FFT spectrogram comprises a series of peaks (P1 to P5 as shown in Figure 5). The peaks P1 to P5 are indicative of the intermediate peaks 406a-d between each signal peak 402a-d and the corresponding signal valley 404a-d. The FFT spectrogram therefore allows the extraction of data from the output of the sensor 102 relating to the intermediate peaks 406a-d.

The blood flow parameter determiner 120 is configured to determine the amplitude of each of the peaks from the FFT spectrogram (i.e. the amplitude of peaks P1, P2, P3, P4, P5 as shown in Figure 5), for example using a peak detection algorithm. The blood flow parameter determiner 120 determines the systolic and diastolic blood pressure based on the determined amplitudes of the peaks from the FFT. The systolic blood pressure can be determined from the ratio of P1/P5. The diastolic blood pressure can be determined from the ratio of P4/P5.

306: The blood flow impairment determiner 122 determines whether blood flow through the blood vessel/AVF is impaired based on the determined blood flow parameters. Specifically, the blood flow impairment determiner 122 determines whether blood flow through the blood vessel/AVF is impaired based on the determined maximum and minimum diameters of the blood vessel/AVF over which the sensor 102 is placed, and the systolic and diastolic blood pressures determined from the data collected by the sensor 102.

Determination of whether blood flow through the blood vessel/AVF is impaired may comprise determining the degree of stenosis (DOS) and/or the blood flow volume (BFV) based on the blood flow parameters determined by the blood flow parameter determiner 120, as described below.

### Degree of Stenosis (DOS)

The degree of stenosis (DOS) is defined as the ratio of the cross sectional area between a normal unaffected blood vessel/AVF and a stenotic region of the blood vessel/AVF: $DOS = \left(1-\frac{{d}^{2}}{{D}^{2}}\right) \times 100 %$

Where d is the blood vessel/AVF diameter at the stenotic region, and D is the diameter of the normal, unaffected AVF.

The above equation may be expressed using the blood vessel/AVF thickness, as below: $DOS = \left(1-\frac{{d}^{2}}{{\left(d+2{h}_{2}\right)}^{2}}\right) \times 100 % = \left(1-\frac{{d}^{2}}{{\left(d+2h-2{h}_{1}\right)}^{2}}\right) \times 100 %$

Where d is the diameter of the stenosis, *h*₁ is the thickness of the blood vessel/AVF wall, *h*₂ is the thickness of the stenotic region, and *h* is the total thickness of the blood vessel wall and the stenotic region (i.e. *h = h*₁ *+ h*₂)*.* These parameters are shown in Figure 6, where 602 is the blood vessel wall and 604 is the stenotic region.

Using the Telegrapher model/equations to relate the hemodynamics of the blood vessel/AVF to strain gauge theory, the total thickness h may be expressed based on the definition of the strain gauge capacitance: $C ≡ \frac{{A}_{max} - {A}_{min}}{SBP - DBP} ≡ \frac{\left(1-{σ}^{2}\right) {πd}_{0}^{3}}{4 hE}$

Where *Aₘₐₓ* is the maximum cross sectional area of the blood vessel/AVF (i.e. $π {\left(\frac{{d}_{max}}{2}\right)}^{2}$), and *Aₘᵢₙ* is the minimum cross sectional area of the blood vessel/AVF (i.e. $π {\left(\frac{{d}_{min}}{2}\right)}^{2}$).

As such, the total thickness, *h*, may be expressed as: $h = \frac{\left(1-{σ}^{2}\right) {πd}_{0}^{3}}{E} \frac{SBP - DBP}{{d}_{max}^{2} - {d}_{min}^{2}}$

Where *σ* is the Poissons ratio of the blood vessel/AVF, *d*₀ is the initial diameter of the blood vessel/AVF (that is, the initial diameter of the blood vessel before creation of the AVF), and E is the Young's modulus of the blood vessel/AVF.

*σ, d*₀ and E are constants that can be determined for the blood vessel/AVF from medical guidelines (for example, the National Kidney Foundation). For example, typically *d*₀ is within a certain range for most patients, specifically 1.5mm to 2.5mm, *E* is approximately 0.5MPa and *σ* is approximately 0.45 to 0.55. Furthermore, the systolic and diastolic blood pressures (SBP and DBP), and the maximum and minimum diameters of the blood vessel/AVF (*dₘₐₓ* and *dₘᵢₙ*) over which the sensor 102 is placed can be determined as described above based on the data collected by the sensor 102. As such, the maximum thickness, *h*, may be determined.

The DOS may therefore be calculated using the below equation: $DOS = \left(1-\frac{{d}^{2}}{{\left(d+2h-2{h}_{1}\right)}^{2}}\right) \times 100 %$

As described above, the diameter of the stenotic region, *d,* may be determined based on the data collected by the sensor 102 (that is, from the signal mean). The thickness of the blood vessel/AVF wall, *h*₁, can be determined from medical guidelines (for example, the National Kidney Foundation). The thickness of the blood vessel/AVF wall typically falls within the range of 0.2mm to 0.4mm for most patients.

### Blood Flow Volume (BFV)

The blood flow volume of the blood vessel/AVF can be determined based on the definition of the strain gauge impedance, *Z*, (equation [1] given below) and the Telegrapher equations (equations [2] and [3] given below). ${Z}_{0} \frac{P \left(z\right)}{Q \left(z\right)} = \frac{{P}_{0}^{+}}{{Q}_{0}^{+}} = \sqrt{\frac{R + jωL}{G + jωC}}$ [1] $p \left(z, t\right) = {P}_{0}^{+} {e}^{- αz} cos \left(-βz+ωt\right)$ [2] $q \left(z, t\right) = {Q}_{0}^{+} {e}^{- αz} cos \left(-βz+ωt\right)$ [3]

Where *Z*₀ is the average impedance of the strain gauge circuit, P(z) is the blood flow pressure, Q(z) is the blood flow volume, *P*₀⁺ is the average blood flow pressure, *Q*₀⁺ is the average blood flow volume, R is the resistance of the strain gauge circuit, L is the inductance of the strain gauge circuit, C is the capacitance of the strain gauge circuit, and G is the conductance of the strain gauge circuit.

Equation [3] may be written with *Z*₀ is the average impedance of the strain gauge circuit, as below: $q \left(z, t\right) = \frac{{P}_{0}^{+}}{{Z}_{0}} {e}^{- αz} cos \left(-βz+ωt\right)$

Assuming that the sensor 102 is positioned as z = 0, the equation becomes: $q \left(0.t\right) = {P}_{0}^{+} cos \left(ωt\right) \sqrt{\frac{G + jωC}{R + jωL}}$

The average blood pressure *P*₀⁺ (or mean blood pressure, MPB) can be calculated based on the systolic and diastolic blood pressures determined from the data collected by the sensor 102 as below: ${P}_{0}^{+} ≡ MBP = \frac{1}{3} SBP + \frac{2}{3} DBP$

As such, the average blood flow volume may be determined using the following equation: ${q}_{avg} = \left|MBP\sqrt{\frac{G + jωC}{R + jωL}}\right|$

Where G, R, C and L may all be determined using the following equations: $R = {C}_{1} \frac{128 η}{{d}_{0}^{4}} , L = {C}_{2} \frac{4 ρ}{{πd}_{0}^{2}} , G = 0 , C = \frac{\left(1-{σ}^{2}\right) {πd}_{0}^{3}}{4 hE}$

Where:

| | |
|---|---|
| *η* | Dynamic viscosity ( $0.035 \frac{g}{cm . s}$) |
| *ρ* | Blood density ( $1.056 \frac{g}{{cm}^{3}}$) |
| σ | Poisson's ratio of blood vessel |
| *ω* | Heart rate frequency |
| *h* | The thickness of the blood vessel wall |
| *E* | Young's elastic modulus of blood vessel |
| *d*₀ | The initial diameter of the blood vessel |
| *W* | Womersley number equal to $W = {d}_{0} \sqrt{\frac{ωρ}{4 η}}$ |
| *C*₁ | Heart rate constant equal to: |
| | *C*₁ = 0.18*W* + 0.45 |
| *C*₂ | Heart rate constant equal to: |
| | *C*₂ = -0.018*W* + 1.39 |

As such, the degree of stenosis (DOS) and/or the blood flow volume (BFV) may be determined based on the blood flow parameters determined by the blood flow parameter determiner 120.

308: Based on the degree of stenosis (DOS) and/or the blood flow volume (BFV), the blood flow impairment determiner 122 may determine the extent of blood flow impairment. This may comprise comparing the determined degree of stenosis to a stenosis threshold and/or comparing the determined blood flow volume to a blood flow volume threshold. Based on the comparison of the determined degree of stenosis to the stenosis threshold and/or the comparison of the determined blood flow volume to the blood flow volume threshold, the blood flow impairment determiner 122 may determine a risk level. The risk level may be one of: high risk, moderate risk or low risk. In one example, the stenosis threshold may be one of substantially 25%, substantially 30% and substantially 35% (i.e. a degree of stenosis of one of substantially 25%, substantially 30%, and substantially 35%), and the blood flow threshold may be one of substantially 600ml/min, substantially 650ml/min, substantially 700ml/min and substantially 750ml/min.

For example, the blood flow determiner 122 may determine that the user is at high risk if the degree of stenosis exceeds the stenosis threshold, and the blood flow volume is lower than the blood flow threshold. The blood flow determiner 122 may determine that the user is at moderate risk if the degree of stenosis exceeds the stenosis threshold and the blood flow volume exceeds the blood flow threshold. The blood flow determiner 122 may determine that the patient is at low risk if the degree of stenosis is below the stenosis threshold and the blood flow volume exceeds the blood flow threshold.

In exemplary arrangements, the blood flow threshold may comprise a first blood flow threshold and a second blood flow threshold, which may be higher than the first blood flow threshold. In such arrangements, the blood flow determiner 122 may determine that the user is at high risk if the degree of stenosis exceeds the stenosis threshold, and the blood flow volume is lower than the first blood flow threshold. The blood flow determiner 122 may determine that the user is at moderate risk if the degree of stenosis exceed the stenosis threshold and the blood flow volume exceeds the second blood flow threshold. The blood flow determiner 122 may determine that the patient is at low risk if the degree of stenosis is below the stenosis threshold and the blood flow volume exceeds the second blood flow threshold. In one example, the stenosis threshold may be substantially 30%, the first blood flow threshold may be substantially 600ml/min and the second blood flow threshold may be substantially 750ml/min.

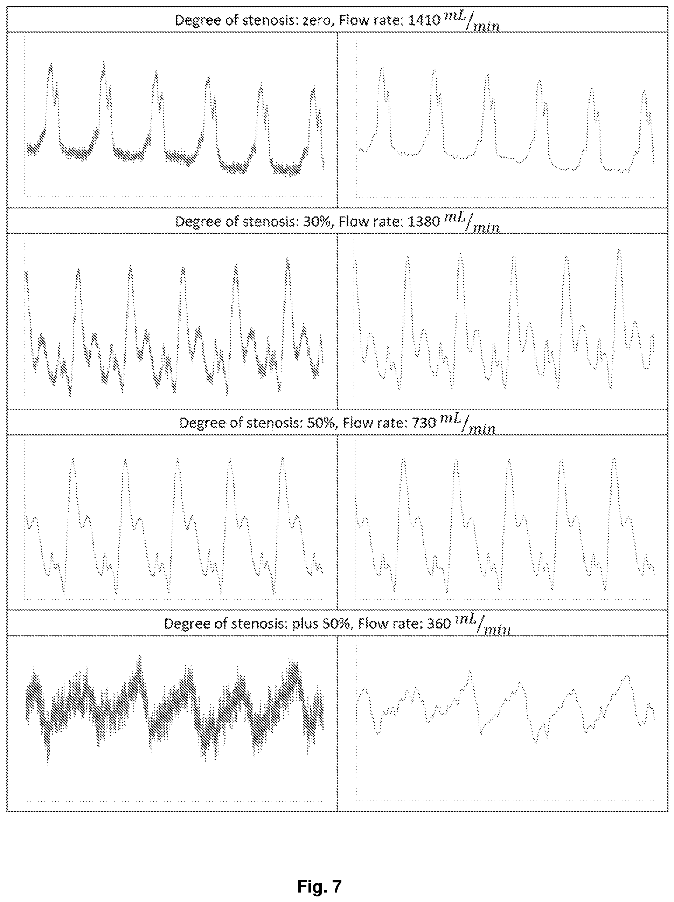

Figure 7 shows a series of output plots produced from data collected by the sensor 102, when placed over a series blood vessels, such as AVFs, with varying degrees of stenoses. The plots in the left column show the unfiltered data collected by the sensor 102, and the plots in the right column show the filtered data (i.e. the data after being processed by the filtering module 118). The plots show the strain measured by the strain gauge on the y-axis and time on the x-axis. Figure 7 shows how the waveforms (and therefore blood flow parameters) may change when stenoses are present, however the skilled person will appreciate that the waveforms shown in Figure 7 are exemplary only and are provided to aid understanding.

310: An indication may be provided to the user indicating whether the blood flow through the blood vessel is impaired. In exemplary arrangements, the indication may comprise a visual indication provided to the user via the display 126 of the controller 104. In alternative arrangements an audio or haptic indication may be provided to the user. The skilled person will be able to envisage alternative indications/indicators.

In exemplary arrangements, the risk level is indicated to the user. In the exemplary arrangement shown in Figure 1, the controller 104 comprises a display 126, and the risk level may be displayed to the user on the display 126. Alternatively, or additionally, one or more of the determined blood parameters, the determined degree of stenosis, and the determined blood flow volume may be indicated to the user (e.g. via the display 126).

An indication of the action that the user should take in response to the determined risk level may also be indicated to the user. For example, if the determined risk level is "high risk", an indication that the user should follow up with a professional may be displayed to the user.

In alternative arrangements, data indicating the determined risk level may be transmitted by the transmitter 112 of the controller 104 to external user equipment, such as a mobile device or the user, and the external user equipment may provide an indication of the risk level to the user (e.g. via a display of the user equipment).

Advantageously, the blood flow monitoring device 100 disclosed herein allows a user to regularly monitor a blood vessel, such as an AVF, outside of a professional setting. The user simply needs to place the sensor 102 against the skin above the AVF and using the method disclosed above, the blood flow monitoring device 100 is able to provide an indication to the user as to the extent of impairment of the AVF and whether further investigation by a professional is needed. As such, the blood flow monitoring device 100 provides a cost effective, quick, and easy to use method for users to monitor their AVFs, especially when compared to traditional methods such as the use of Doppler ultrasound.

It will be appreciated by the person of skill in the art that various modifications may be made to the above described embodiments.

The word "exemplary" is used herein to mean "an example". Any embodiment described herein as "exemplary" is not necessarily to be construed as preferred or advantageous over other embodiments.

## Claims

1. A blood flow monitoring device (100) comprising:
a sensor (102) configured to be placed against skin (108) of a user and to collect data indicative of movement of the skin (108) caused by blood flow through a blood vessel in a region under the skin, wherein an output of the sensor comprises a waveform comprising a series of peaks (402a-d) and valleys (404a-d); and
a controller (104) comprising:
a blood flow parameter determiner (120) configured to determine, based on the waveform output by the sensor (102), a systolic blood pressure, a diastolic blood pressure, a maximum diameter of the blood vessel and a minimum diameter of the blood vessel, wherein the systolic blood pressure is determined based on an average height of the peaks of the waveform from the x-axis, the diastolic blood pressure is determined based on an average height of the valleys of the waveform from the x-axis, the maximum diameter of the blood vessel is determined based on the average height of the peaks from a signal mean of the waveform, and the minimum diameter of the blood vessel is determined based on the average height of the valleys from the signal mean of the waveform, and
a blood flow impairment determiner (122) configured to determine a degree of stenosis and a blood flow volume based on the determined maximum diameter of the blood vessel, the determined minimum diameter of the blood vessel, the determined systolic blood pressure and the determined diastolic blood pressure.

2. A blood flow monitoring device (100) according to claim 1, wherein the sensor (102) comprises a sensing element configured to deform on movement of the skin (108) of the user, and wherein deformation of the sensing element changes an electrical property of the sensor (102).

3. A blood flow monitoring device (100) according to claim 1 or 2, wherein the sensor (102) comprises a strain gauge.

4. A blood flow monitoring device (100) according to claim 3, wherein the data indicative of movement of the skin (108) of the user comprises data indicative of strain applied to the strain gauge by movement of the skin (108).

5. A blood flow monitoring device (100) according to any preceding claim, further comprising a plurality of sensors (102) configured to be placed against the skin (108) of the user, wherein each sensor (102) is configured to collect data indicative of movement of the skin (108).

6. A blood flow monitoring device (100) according to any preceding claim, wherein the blood flow impairment determiner is further configured to determine an extent of blood flow impairment based on the degree of stenosis and the blood flow volume.

7. A blood flow monitoring device (100) according to claim 6, wherein the blood flow impairment determiner is configured to compare the degree of stenosis to a stenosis threshold and the blood flow volume to a blood flow volume threshold to determine the extent of the blood flow impairment.

8. A blood flow monitoring device (100) according to claim 7, wherein the blood flow impairment determiner is configured to determine a risk level comprising one of a high risk, a moderate risk and a low risk, based on the comparison of the degree of stenosis with the stenosis threshold and the comparison of the blood flow volume with the blood flow volume threshold.

9. A blood flow monitoring (100) device according to any preceding claim, further comprising an indicator configured to provide an indication to the user indicating whether the blood flow through the blood vessel is impaired.

## Patentansprüche

1. Blutflussüberwachungsvorrichtung (100), umfassend:
einen Sensor (102), der so konfiguriert ist, dass er auf der Haut (108) eines Benutzers platziert wird und Daten erfasst, die auf eine Bewegung der Haut (108) hinweisen, die durch den Blutfluss durch ein Blutgefäß in einem Bereich unter der Haut verursacht wird, wobei eine Ausgabe des Sensors eine Wellenform umfasst, die eine Reihe von Spitzen (402a-d) und Tälern (404a-d) umfasst; und eine Steuereinheit (104), die Folgendes umfasst:
ein Gerät zur Bestimmung von Blutflussparametern (120), das so konfiguriert ist, dass es anhand der vom Sensor (102) ausgegebenen Wellenform einen systolischen Blutdruck, einen diastolischen Blutdruck, einen maximalen Durchmesser des Blutgefäßes und einen minimalen Durchmesser des Blutgefäßes bestimmt, wobei der systolische Blutdruck anhand einer durchschnittlichen Höhe der Wellenformspitzen relativ zur x-Achse bestimmt wird, der diastolische Blutdruck anhand einer durchschnittlichen Höhe der Wellenformtäler relativ zur x-Achse bestimmt wird, der maximale Durchmesser des Blutgefäßes anhand der durchschnittlichen Höhe der Wellenformspitzen relativ zum Signalmittelwert bestimmt wird und der minimale Durchmesser des Blutgefäßes anhand der durchschnittlichen Höhe der Wellenformtäler relativ dem Signalmittelwert bestimmt wird, und
ein Gerät zur Bestimmung der Durchblutungsbeeinträchtigung (122), das so konfiguriert ist, dass es einen Stenosegrad und ein Blutflussvolumen anhand des bestimmten maximalen Durchmessers des Blutgefäßes, des bestimmten minimalen Durchmessers des Blutgefäßes, des bestimmten systolischen Blutdrucks und des bestimmten diastolischen Blutdrucks bestimmt.

2. Blutflussüberwachungsvorrichtung (100) nach Anspruch 1, wobei der Sensor (102) ein Sensorelement umfasst, das so konfiguriert ist, dass es sich bei Bewegung der Haut (108) des Benutzers verformt, und wobei die Verformung des Sensorelements eine elektrische Eigenschaft des Sensors (102) verändert.

3. Blutflussüberwachungsvorrichtung (100) nach Anspruch 1 oder 2, wobei der Sensor (102) einen Dehnungsmessstreifen umfasst.

4. Blutflussüberwachungsvorrichtung (100) nach Anspruch 3, wobei die Daten, die auf eine Bewegung der Haut (108) des Benutzers hinweisen, Daten umfassen, die auf die Dehnung hinweisen, die durch die Bewegung der Haut (108) auf den Dehnungsmessstreifen ausgeübt wird.

5. Blutflussüberwachungsvorrichtung (100) nach einem vorstehenden Anspruch, weiter umfassend eine Vielzahl von Sensoren (102), die so konfiguriert sind, dass sie auf der Haut (108) des Benutzers platziert werden, wobei jeder Sensor (102) so konfiguriert ist, dass er Daten erfasst, die auf eine Bewegung der Haut (108) hinweisen.

6. Blutflussüberwachungsvorrichtung (100) nach einem vorstehenden Anspruch, wobei das Gerät zur Bestimmung der Durchblutungsbeeinträchtigung weiter so konfiguriert ist, dass es ein Ausmaß der Blutflussbeeinträchtigung anhand des Stenosegrades und des Blutflussvolumens bestimmt.

7. Blutflussüberwachungsvorrichtung (100) nach Anspruch 6, wobei das Gerät zur Bestimmung der Durchblutungsbeeinträchtigung so konfiguriert ist, dass es den Stenosegrad mit einem Stenoseschwellenwert und das Blutflussvolumen mit einem Blutflussvolumenschwellenwert vergleicht, um das Ausmaß der Blutflussbeeinträchtigung zu bestimmen.

8. Blutflussüberwachungsvorrichtung (100) nach Anspruch 7, wobei das Gerät zur Bestimmung der Durchblutungsbeeinträchtigung so konfiguriert ist, dass es anhand des Vergleichs des Stenosegrades mit dem Stenoseschwellenwert und des Vergleichs des Blutflussvolumens mit dem Blutflussvolumenschwellenwert ein Risikoniveau bestimmt, umfassend eines von hohem Risiko, mittlerem Risiko und niedrigem Risiko.

9. Blutflussüberwachungsvorrichtung (100) nach einem vorstehenden Anspruch, weiter umfassend einen Indikator, der so konfiguriert ist, dass er dem Benutzer eine Angabe bereitstellt, die angibt, ob der Blutfluss durch das Blutgefäß beeinträchtigt ist.

## Revendications

1. Dispositif de surveillance du flux sanguin (100) comprenant :
un capteur (102) configuré pour être placé contre la peau (108) d'un utilisateur et pour recueillir des données indicatives du mouvement de la peau (108) causé par le flux sanguin dans un vaisseau sanguin situé sous la peau, dans lequel l'émission du capteur comprend une forme d'onde constituée d'une série de pics (402a-d) et de creux (404a-d) ; et un dispositif de commande (104) comprenant :
un dispositif de détermination des paramètres de flux sanguin (120) configuré pour déterminer, à partir de l'émission de forme d'onde du capteur (102), la pression artérielle systolique, la pression artérielle diastolique, le diamètre maximal et le diamètre minimal du vaisseau sanguin, dans lequel la pression artérielle systolique est déterminée à partir de la hauteur moyenne des pics de la forme d'onde par rapport à l'axe des abscisses, la pression artérielle diastolique est déterminée à partir de la hauteur moyenne des creux de la forme d'onde par rapport à l'axe des abscisses, le diamètre maximal du vaisseau sanguin est déterminé à partir de la hauteur moyenne des pics d'un signal moyen de la forme d'onde, et le diamètre minimal du vaisseau sanguin est déterminé à partir de la hauteur moyenne des creux du signal moyen de la forme d'onde, et
un dispositif de détermination d'altération du flux sanguin (122) configuré pour déterminer un degré de sténose et un volume de flux sanguin en fonction du diamètre maximal déterminé du vaisseau sanguin, du diamètre minimal déterminé du vaisseau sanguin, de la pression artérielle systolique déterminée et de la pression artérielle diastolique déterminée.

2. Dispositif de surveillance du flux sanguin (100) selon la revendication 1, dans lequel le capteur (102) comprend un élément de détection configuré pour se déformer lors du mouvement de la peau (108) de l'utilisateur, et dans lequel la déformation de l'élément de détection modifie une propriété électrique du capteur (102).

3. Dispositif de surveillance du flux sanguin (100) selon la revendication 1 ou 2, dans lequel le capteur (102) comprend une jauge de contrainte.

4. Dispositif de surveillance du flux sanguin (100) selon la revendication 3, dans lequel les données indicatives du mouvement de la peau (108) de l'utilisateur comprennent des données indicatives de la contrainte appliquée à la jauge de contrainte par le mouvement de la peau (108).

5. Dispositif de surveillance du flux sanguin (100) selon une quelconque revendication précédente, comprenant en outre une pluralité de capteurs (102) configurés pour être placés contre la peau (108) de l'utilisateur, dans lequel chaque capteur (102) est configuré pour collecter des données indicatives du mouvement de la peau (108).

6. Dispositif de surveillance du flux sanguin (100) selon une quelconque revendication précédente, dans lequel le dispositif de détermination de l'altération du flux sanguin est en outre configuré pour déterminer l'étendue de l'altération du flux sanguin en fonction du degré de sténose et du volume du flux sanguin.

7. Dispositif de surveillance du flux sanguin (100) selon la revendication 6, dans lequel le dispositif de détermination de l'altération du flux sanguin est configuré pour comparer le degré de sténose à un seuil de sténose et le volume du flux sanguin à un seuil de volume du flux sanguin afin de déterminer l'étendue de l'altération du flux sanguin.

8. Dispositif de surveillance du flux sanguin (100) selon la revendication 7, dans lequel le dispositif de détermination de l'altération du flux sanguin est configuré pour déterminer un niveau de risque comprenant un risque élevé, un risque modéré et un risque faible, sur la base de la comparaison du degré de sténose avec le seuil de sténose et de la comparaison du volume de flux sanguin avec le seuil de volume de flux sanguin.

9. Dispositif de surveillance du flux sanguin (100) selon une quelconque revendication précédente, comprenant en outre un indicateur configuré pour fournir à l'utilisateur une indication indiquant si le flux sanguin à travers le vaisseau sanguin est altéré.
